# FASCICULE DE BREVET EUROPEEN

(11) **EP 0 323 874 B1**
(45) Date de publication et mention de la délivrance du brevet: **07.04.1993**
(21) Numéro de dépôt: 89200025.8
(22) Date de dépôt: 06.01.1989
(51) Int. Cl.: B65D 83/14, A61L 9/12

(54) **Dispositif formant capuchon protecteur d'un dispositif de stockage et de pulvérisation de produits destinés à être distribués dans l'atmosphère tels que les parfums et insecticides, et dispositif de stockage et de pulvérisation pourvu d'un tel dispositif formant capuchon protecteur**
Mit einer Schutzkappe versehener Behälter zum Versprühen von Produkten, wie Parfüms oder Insektiziden, sowie Schutzkappe für einen solchen Behälter
Spray device for products to be dispensed into the atmosphere, such as parfumes and insecticides, and protective cap therefor

(30) Priorité: 08.01.1988 FR 8800149
(43) Date de publication de la demande: 12.07.1989
(73) Titulaire: Douwe Egberts Koninklijke Tabaksfabriek- Koffiebranderijen-Theehandel N.V., 3532 AA Utrecht (NL)
(72) Inventeur: Bonjour, Georges, F-69450 Saint Cyr au Mont d'Or France (FR)
(74) Mandataire: Smulders, Theodorus A.H.J., Ir.

(56) Documents cités:
- DE-A- 1 642 020
- US-A- 2 383 960
- US-A- 3 156 382
- US-A- 3 262 607
- US-A- 3 940 024
- US-A- 3 972 473
- US-A- 4 341 348

## Description

L'invention concerne essentiellement un dispositif formant capuchon protecteur d'un dispositif de stockage et de pulvérisation de produits destinés à être distribués dans l'atmosphère tels que les parfums et insecticides, et dispositif de stockage de pulvérisation pourvu d'un tel dispositif formant capuchon protecteur.

On connaît déjà par le document US-A-4 341 348 un dispositif formant capuchon protecteur (26, 28) d'un dispositif (12) de stockage et de pulvérisation de produits destinés à être distribués dans l'atmosphère, tels que parfums et insecticides, habituellement communément appelé bombe aérosol. Ces dispositifs 12 de stockage et de pulvérisation sont constitués d'un réservoir de stockage proprement dit (12) contenant les produits destinés à être distribués dans l'atmosphère, et un dispositif d'éjection (16, 18) desdits produits. Le capuchon est formé d'une coque protectrice (26, 28) pour le dispositif d'éjection (16, 18) présentant une paroi latérale (26, 28), par exemple sensiblement cylindrique, ayant une extrémité inférieure (32) destinée à venir coopérer avec le réservoir de stockage (12) ainsi qu'une extrémité supérieure (30) sensiblement fermée constituant une paroi de sommet de la coque protectrice.

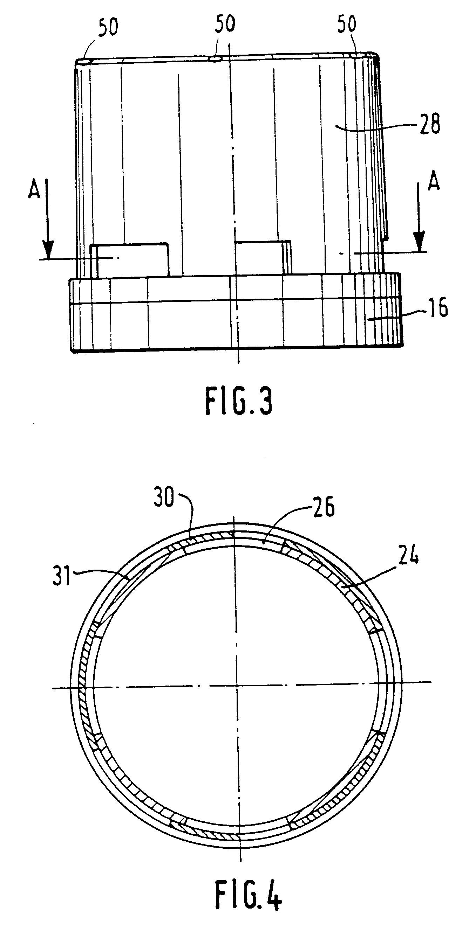

Un moyen (46, 48) absorbant ledit produit est disposé transversalement à l'intérieur de la coque protectrice (26, 28, 30) permettant l'absorption et la réémission dudit produit dans l'atmosphère. Des moyens de diffusion (38, 42) sont prévus pour permettre une diffusion dudit produit à l'extérieur de la coque constituant le capuchon protecteur. Ces moyens de diffusion (38, 42) comprennent une ou plusieurs ouvertures (38) prévues dans la paroi latérale (28) au niveau de l'extrémité inférieure (32) pour créer une circulation d'air à l'intérieur de la coque protectrice comme symbolisé par les flèches à la figure 3.

Le moyen absorbant (46, 48) comprend un orifice axial (50, 52) permettant le libre passage, avec jeu, du dispositif d'éjection (16, 18). Le dispositif d'éjection (16, 18) se trouve en regard du moyen absorbant (46, 48) au moins pour certaines positions du capuchon (26, 28, 30) montées sur le réservoir (12). En outre, un moyen (18) est prévu, qui permet d'actionner le dispositif d'éjection (16) depuis l'extérieur.

Dans la réalisation décrite dans ce document, le moyen d'actionnement (18) est constitué par un élément piston spécial qui vient s'emmancher sur la tige (16) de la valve constituant le dispositif d'éjection du produit contenu dans le réservoir (12). Cet élément piston (18) est d'une longueur suffisante pour dépasser suffisamment à l'extérieur du capuchon en passant au travers d'un orifice axial (44) prévu dans la paroi de sommet (30), pour permettre un actionnement de la valve de l'extérieur comme montré clairement à la figure 3.

Il est également prévu dans la paroi latérale (28) deux ouvertures (40) diamétralement opposées, permettant une diffusion directe dans l'atmosphère des produits éjectés du réservoir (12) en passant dans des passages (54, 56) ménagés dans le moyen absorbant (46, 48). En pratique, ce moyen absorbant (46, 48) est réalisé en deux demi-disques (46 et 48) comportant des découpes adéquates pour définir l'orifice axial (52) et les canaux transversaux (54, 56).

On conçoit qu'avec un tel dispositif, il est nécessaire de réaliser un moyen spécial (18) d'actionnement du dispositif d'éjection (16). En outre, il est nécessaire de réaliser le tampon absorbant sous la forme de deux disques indépendants (46, 48) qu'il faut disposer selon une position précise à l'intérieur du capuchon pour que les canaux (54, 56) soient exactement en coïncidence avec les ouvertures (40) diamétralement opposées. Egalement, il en est de même pour le moyen d'actionnement (18) qui comporte des évidements spéciaux pour permettre la pulvérisation latérale des produits éjectés par la valve (16), comme cela est clairement compréhensible et symbolisé par des traits mixtes aux figures 3 à 5.

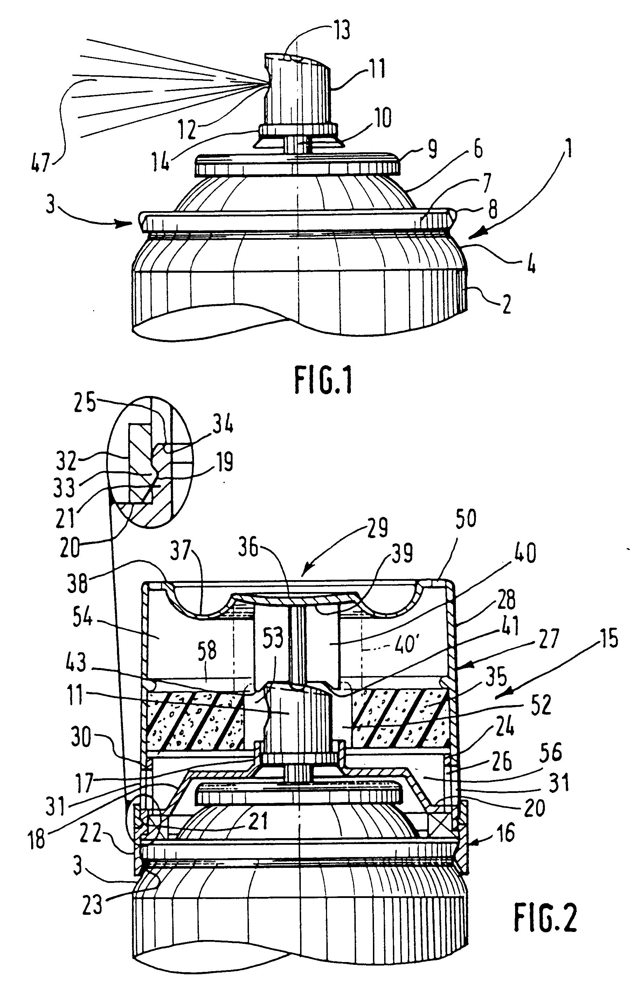

Enfin, il est essentiel d'observer que le moyen absorbant (46, 48) est appliqué contre la paroi de sommet (30), directement contre les orifices (42) (voir figures 1, 3 et 4).

Cette structure limite grandement la circulation de l'air dans le sens des flèches indiquées (figure 3), en limitant ainsi la diffusion des produits absorbés par le moyen absorbant (46, 48) et qui sont destinés à être réémis dans l'atmosphère.

Ce dispositif est donc compliqué et relativement coûteux à fabriquer car il utilise un moyen d'actionnement de la valve spécifique alors qu'il est absolument impératif de concevoir un dispositif formant capuchon protecteur pouvant être utilisé sur n'importe quel dispositif de stockage et de pulvérisation dans le commerce.

De même, le document FR-A-2 556 242 décrit un dispositif diffuseur de produits d'ambiance à double action, immédiate et différée, ainsi qu'un conditionnement aérosol équipé d'un tel diffuseur, d'une réalisation complexe par la nécessité de réaliser deux tubulures axiales disposées perpendiculairement dans une pièce d'assemblage (17). La structure de ce dispositif étant complexe, le coût de fabrication en est élevé. En outre, par le fait que le tampon absorbant (7) est appliqué directement contre les orifices (8) de la paroi de sommet (16), la circulation de l'air est également limitée comme dans le dispositif précédent. Ce dispositif d'assemblage (17) sert de moyen d'actionnement de la valve en venant coopérer avec la paroi de sommet (16) qui est en partie déformable par pression, en permettant ainsi un actionnement de la valve de l'extérieur.

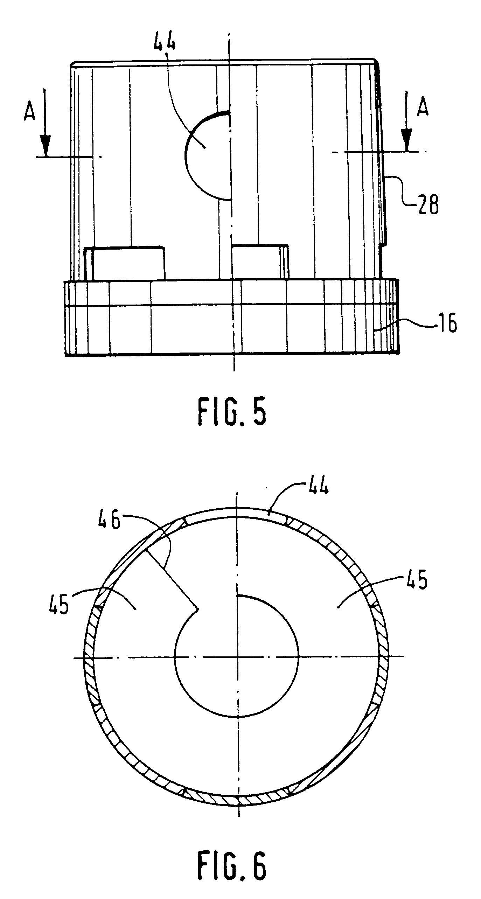

Le document US-A-3 972 473 décrit encore un dispositif formant capuchon protecteur (22) d'un dispositif (12) de stockage et de pulvérisation de produits destinés à être distribués dans l'atmosphère, tels que parfums, etc., habituellement communément appelés bombe aérosol, qui peut être monté sur n'importe quel type de bombe aérosol disponible dans le commerce. La paroi supérieure ou de sommet comporte une zone déformable (34) s'appliquant directement sur le dispositif d'éjection (16, 18). Selon diverses variantes de réalisation représentées aux figures 4 à 6, on peut prévoir un moyen absorbant (50 ou 52), disposé soit au voisinage immédiat du système d'éjection (16, 18) (figures 4 et 6), soit sous ce dispositif d'éjection (16, 18) dans un logement annulaire (44) prévu au sommet du réservoir autour du dispositif d'éjection et qui est destiné à recueillir dans ce dernier cas les gouttes de produits pulvérisés émises depuis le dispositif de pulvérisation (16, 18) (voir figure 5).

Il est clair que dans le cas où le tampon (52) est disposé dans un logement annulaire (44) de l'embouchure du réservoir (12), aucun courant d'air ne passe au travers du moyen tampon (52) de sorte que la diffusion sera extrêmement réduite. Il en est de même dans le cas des variantes de réalisation des figures 4 et 6 puisque le chemin direct de passage de l'air entre les ouvertures latérales (28) et les orifices de sommet (32) est extérieur au moyen absorbant (50). En outre, ces variantes de réalisation des figures 4 à 6 exigent des adaptations particulières du réservoir, ou du capuchon protecteur, avec l'inconvénient majeur d'une diffusion extrêmement réduite.

Le document US-A-4 084 732 décrit encore un dispositif formant capuchon protecteur d'une structure très complexe qui permet une éjection immédiate ou différée dans le temps, selon certaines orientations du capuchon protecteur relativement à l'orifice d'éjection du dispositif d'éjection de la bombe aérosol (12). Ici encore, il est nécessaire d'utiliser un moyen d'actionnement (18) spécifique, accessible de l'extérieur. En outre, le moyen absorbant (40) est disposé dans la partie inférieure du capuchon protecteur en s'appliquant immédiatement contre des ouvertures latérales (34). Ce moyen absorbant (40) est imbibé de produits grâce à la prévision d'un segment du matériau absorbant (42) disposé verticalement et qui est capable, dans la position de la figure 2, de se trouver en regard de l'orifice d'émission du dispositif d'éjection. Le produit projeté sur ce segment (42) est transféré par effet mêche du matériau absorbant (40). On comprend qu'avec un tel dispositif, il soit nécessaire d'éjecter des quantités considérables de produits pour imprégner suffisamment le moyen absorbant (40). En outre, le moyen absorbant (40) s'appliquant directement contre les ouvertures (34), la circulation d'air est limitée comme dans les documents précédemment énoncés.

On peut encore citer le document US-A-3 262 607 qui décrit un capuchon protecteur (32) comportant une zone déformable pouvant être réalisée selon différentes manières représentées aux figures 1 à 7. Aucun moyen absorbant n'est prévu. Enfin, le document FR-A-1 296 098 décrit encore un système de valve commandée par une partie déformable (24′), sans l'emploi d'un moyen absorbant. La réalisation est également très complexe.

La présente invention a pour but de résoudre le nouveau problème technique consistant en la fourniture d'un dispositif formant capuchon protecteur pour dispositif de stockage et de distribution de produits actifs dans l'atmosphère, tels que parfums et insecticides, habituellement dénommés bombe aérosol, d'une conception particulièrement simple, exigeant un faible coût de fabrication, permettant de réaliser soit une éjection instantanée dans l'atmosphère, soit une éjection différée, par diffusion continue, contrôlée et ce pendant plusieurs heures, voire même pendant au moins un jour.

La présente invention a encore pour but de résoudre un nouveau problème technique consistant en la fourniture d'un dispositif formant capuchon protecteur pour "bombe aérosol", du type à moyen absorbant à l'intérieur du capuchon protecteur, qui permet une circulation d'air améliorée, en aboutissant ainsi à une meilleure diffusion des produits absorbés par le moyen absorbant, en ne rendant ainsi nécessaire l'imprégnation par le moyen absorbant que d'une faible quantité de produits à chaque éjection.

La présente invention a encore pour but de résoudre le nouveau problème technique consistant en la fourniture d'un dispositif formant capuchon protecteur du type tampon absorbant, qui présente une circulation de l'air suffisante pour permettre la diffusion de produits secs, en particulier d'un parfum sec, c'est-à-dire sans solvant, contrairement aux dispositifs antérieurs qui nécessitent la présence d'un solvant pour une meilleure imprégnation, ce qui diminue l'efficacité du produit, ou nécessite des manipulations spéciales avant l'incorporation dans la bombe aérosol.

Tous ces nouveaux problèmes techniques sont résolus pour la première fois par la présente invention d'une manière satisfaisante, utilisable à l'échelle industrielle, en aboutissant ainsi à la fourniture d'un dispositif très performant, moins coûteux, ayant une efficacité prolongée dans le temps par l'emploi de quantités plus faibles à chaque éjection.

Ainsi, selon un premier aspect, la présente invention fournit un dispositif formant capuchon protecteur d'un dispositif de stockage et de pulvérisation de produits destinés à être distribués dans l'atmosphère, tels que parfums et insecticides, habituellement communément appelé bombe aérosol, constitué d'un réservoir de stockage proprement dit desdits produits destinés à être distribués dans l'atmosphère et d'un dispositif d'éjection desdits produits, ledit capuchon étant formé d'une coque protectrice pour le dispositif d'éjection présentant une paroi latérale, par exemple sensiblement cylindrique, ayant une extrémité inférieure destinée à venir coopérer avec ledit réservoir de stockage ainsi qu'une extrémité supérieure sensiblement fermée constituant une paroi de sommet de ladite coque protectrice, un moyen absorbant ledit produit, essentiellement en forme de disque et, s'étendant jusqu'à la paroi latérale, étant disposé transversalement à l'intérieur de ladite coque protectrice permettant l'absorption et la réémission dudit produit dans l'atmosphère, des moyens de diffusion étant prévus pour permettre une diffusion dudit produit à l'extérieur de ladite coque protectrice, ces moyens de diffusion comprenant une ou plusieurs ouvertures prévues dans la paroi latérale au niveau de ladite extrémité inférieure, ainsi que un ou plusieurs orifices au niveau de ladite extrémité supérieure, pour créer une circulation d'air à l'intérieur de ladite coque protectrice et à travers le moyen absorbant, et un moyen permettant l'actionnement du dispositif d'éjection de l'extérieur du capuchon, caractérisé en ce que ledit moyen absorbant en forme de disque est disposé à un niveau intermédiaire entre l'extrémité inférieure et l'extrémité supérieure de la coque protectrice à l'intérieur de ladite coque protectrice, à distance de la paroi de sommet de la coque protectrice en définissant ainsi une chambre de diffusion à l'intérieur de ladite coque protectrice entre le moyen absorbant et la paroi de sommet, en obtenant ainsi une amélioration de la circulation d'air et de la surface d'échange à l'intérieur de ladite coque protectrice, résultant en une amélioration de la diffusion globale desdits produits.

Selon un mode de réalisation particulier du dispositif formant capuchon protecteur selon l'invention, le moyen absorbant précité comprend un orifice axial permettant le libre passage, avec jeu, du dispositif d'éjection, ledit dispositif d'éjection se trouvant en regard dudit moyen absorbant au moins pour certaines positions du capuchon monté sur le réservoir, ledit dispositif étant caractérisé en ce que la chambre de diffusion communique avec l'orifice axial du moyen absorbant.

Selon un mode de réalisation avantageux du dispositif formant capuchon protecteur selon l'invention, celui-ci comprend des moyens d'arrêt du moyen absorbant en position intermédiaire.

Selon une caractéristique particulière du dispositif formant capuchon protecteur selon l'invention, le moyen permettant d'actionner, de l'extérieur de la coque protectrice, le dispositif d'éjection, est constitué par une zone déformable de la coque protectrice. Cette zone déformable est avantageusement située au-dessus du dispositif d'éjection et comprend un prolongement axial vers le bas se terminant au-dessus du dispositif d'éjection, en position montée du dispositif formant capuchon protecteur.

Selon un mode de réalisation particulièrement avantageux du dispositif formant capuchon protecteur selon l'invention, celui-ci est caractérisé en ce qu'il est formé d'une première partie fixée de manière réversible ou non sur la partie supérieure du réservoir, la coque protectrice étant accouplable de préférence de manière réversible à cette première partie et pouvant de préférence pivoter autour de celle-ci.

Selon encore une caractéristique particulière, la première partie précitée présente la forme d'une bague surmontée d'une paroi de forme cylindrique présentant des ouvertures à intervalles réguliers, la coque protectrice est fixée sur ladite bague de manière que la zone inférieure de la coque protectrice comportant les ouvertures précitées soit emmanchée sur la paroi de la bague de manière que les ouvertures de la paroi de la bague et celles de la zone inférieure de la coque protectrice puissent être amenées en coïncidence ou se superposer par rotation de la coque protectrice autour de la bague.

On peut prévoir avantageusement que les fixations de la première partie et de la coque protectrice sont assurées par encliquetage.

Selon une autre caractéristique avantageuse, la force de maintien de la première partie à la partie supérieure du réservoir est supérieure à la force de maintien de la coque protectrice sur la première partie.

Selon un autre mode de réalisation particulier, les moyens d'arrêt précités du moyen absorbant comprennent des premiers moyens d'arrêt contre un déplacement vers le haut et des seconds moyens d'arrêt contre un déplacement vers le bas.

Selon une réalisation particulière, les moyens d'arrêt contre un déplacement vers le haut comprennent une extension du prolongement axial vers le bas de la paroi de sommet se trouvant au-dessus du moyen absorbant et remplissant la fonction de moyen d'actionnement du dispositif d'éjection.

Selon une autre variante de réalisation, les premiers moyens d'arrêt contre un déplacement vers le haut comprennent un épaulement annulaire radialement saillant vers l'intérieur de la coque protectrice.

Selon une autre réalisation particulière, les seconds moyens d'arrêt contre un déplacement vers le bas sont constitués par la paroi de la première partie qui vient s'emmancher à l'intérieur du capuchon protecteur.

Selon un autre mode de réalisation particulier, on pourrait prévoir que la coque protectrice soit pourvue d'une ouverture à hauteur du cône d'éjection, lorsque cette coque est montée sur le réservoir, et le moyen absorbant est interrompu à ce niveau de manière à permettre le passage dudit cône.

Selon un deuxième aspect, la présente invention couvre également un dispositif de stockage et de pulvérisation de produits destinés à être distribués dans l'atmosphère, tels que parfums et insecticides, constitué d'un réservoir de stockage et d'un dispositif d'éjection, caractérisé en ce qu'il est pourvu d'un dispositif formant capuchon protecteur tel que précédemment défini.

On obtient ainsi tous les avantages techniques déterminants précédemment énoncés, à savoir une amélioration radicale de la circulation de l'air à l'intérieur du dispositif formant capuchon protecteur, une amélioration également radicale de la surface d'échange entre le moyen absorbant et l'air, résultant en une amélioration décisive de la diffusion totale des produits. En outre, on peut observer que ce dispositif est particulièrement simple et d'un faible coût de fabrication.

Par ailleurs, et ceci constitue un avantage tout à fait inattendu de l'invention, une amélioration de la circulation de l'air, ainsi que de la surface d'échange permet d'utiliser les produits secs, c'est-à-dire sans solvant, et ceci constitue un progrès technique considérable particulièrement en ce qui concerne les parfums. Ceci rend possible l'utilisation par exemple de 1 % de parfums dans un gaz propulseur habituel. Il peut s'agir par exemple du butane. En outre, il est ainsi possible d'éjecter des quantités extrêmement faibles de produits qui sont diffusés en continu pendant une période de temps équivalente ou même plus grande.

D'autres buts, caractéristiques et avantages de l'invention apparaîtront clairement à la lumière de la description explicative qui va suivre faite en référence aux dessins annexés représentant un mode de réalisation actuellement préféré de l'invention donné simplement à titre d'illustration et qui ne saurait donc en aucune façon limiter la portée de l'invention. Dans les dessins :
- la figure 1 est une vue de face avec arrachement partiel, de la partie supérieure d'un dispositif de stockage et de pulvérisation de produits destinés à être distribués dans l'atmosphère, habituellement communément appelé "bombe aérosol", sans capuchon de protection ;
- la figure 2 est une vue similaire à la figure 1 mais avec la présence d'un dispositif formant capuchon protecteur selon l'invention, représenté en coupe axiale longitudinale selon un plan de symétrie, en position de diffusion ;
- la figure 3 représente une vue de face du dispositif formant capuchon protecteur selon l'invention en position de diffusion (demi-vue de droite) et en position fermée (demi-vue de gauche) ;
- la figure 4 est une vue en coupe transversale selon la ligne de trace A-A de la figure 3 ;
- la figure 5 représente une vue de face d'un deuxième mode de réalisation d'un dispositif formant capuchon protecteur selon l'invention ; et
- la figure 6 est une vue en coupe transversale selon la ligne de trace A-A de la figure 5.

En se référant maintenant aux figures 1 et 2, un dispositif 1 de stockage et de pulvérisation de produits 47 destinés à être pulvérisés dans l'atmosphère, habituellement communément appelé "bombe aérosol", est constitué d'un réservoir 2 de stockage proprement dit des produits destinés à être distribués dans l'atmosphère ayant une partie supérieure 3 formée d'un premier rétrécissement 4 puis d'un méplat 5 et d'un second rétrécissement 6 en forme de dôme tronqué au sommet. Le méplat 5 supporte une coupelle 7 dont les bords arrondis 8 débordent légèrement par rapport à la partie supérieure du rétrécissement 4 en formant ainsi une nervure ou tenon dont le rôle apparaîtra ultérieurement.

Le sommet du dôme 6 supporte une autre coupelle 9 ouverte au centre, ce qui permet le passage de la tige 10 reliant l'intérieur du réservoir 2 au bouton-poussoir 11 bien connu dans l'art avec son évasement 12 au niveau de la sortie du gaz propulsant le produit actif, tel qu'un parfum ou un insecticide, et une gouttière 13 ou méplat au sommet afin de faciliter l'exercice de la pression.

La base du bouton 11 repose sur une coupelle 14 de retenue du fluide éventuellement condensé en liquide lors de l'éjection.

Dans le mode de réalisation du dispositif formant capuchon protecteur selon l'invention représenté aux figures 2 à 4, et qui porte le numéro de référence général 15, ce capuchon est formé d'une coque protectrice 27 pour le dispositif d'éjection 10, 11 présentant une paroi latérale 28, par exemple sensiblement cylindrique comme représenté,étant donné que les bombes aérosols sont habituellement de forme cylindrique, ayant une extrémité inférieure 30-32 destinée à venir coopérer avec le réservoir de stockage 2, ainsi qu'une extrémité supérieure 29 sensiblement fermée constituant une paroi de sommet 36-37-38 de la coque protectrice 27. Par exemple, cette paroi de sommet est formée, en partant du centre vers la périphérie, d'une zone concave 36 bordée par une gouttière circulaire 37 puis d'un rebord plat 38 constituant le bord de l'extrémité supérieure 29.

Un moyen 35 absorbant les produits destinés à être distribués dans l'atmosphère est disposé transversalement à l'intérieur de la coque protectrice 27, permettant l'absorption et la réémission du produit dans l'atmosphère. Des moyens de diffusion 26, 36 sont prévus pour permettre une diffusion dudit produit à l'extérieur de ladite coque protectrice 27.

Ces moyens de diffusion 26, 36 comprennent :
- une ou plusieurs ouvertures 26 prévues dans la paroi latérale 28 au niveau de l'extrémité inférieure 30, comme cela est clairement visible aux figures 2 à 4 ; ainsi que
- un ou plusieurs orifices 50 au niveau de ladite extrémité supérieure 29, par exemple dans le rebord plat 38, ces orifices 50 pouvant déborder dans la paroi latérale 28, comme cela est clairement visible à la figure 2. Ces orifices 50 peuvent être disposés décalés par rapport aux ouvertures 31, comme cela est également visible à la figure 3.

La combinaison de ces orifices 50 et des ouvertures 31 permet de créer une circulation d'air à l'intérieur de la coque protectrice 27.

Le moyen absorbant 35 comprend un orifice axial 52 permettant le libre passage, avec jeu, du dispositif d'éjection 11, ledit dispositif d'éjection 11 se trouvant en regard du moyen absorbant 35 au moins pour certaines positions du capuchon 15 monté sur le réservoir 2, comme cela est également visible à la figure 2.

Un moyen d'actionnement 39, 40 du dispositif d'éjection 11 est également prévu.

Selon l'invention, ce dispositif formant capuchon protecteur 15 est caractérisé en ce que le moyen absorbant 35 est disposé à un niveau intermédiaire à l'intérieur de la coque protectrice 27, à distance de la paroi de sommet 29 (ou 36-37-38) de la coque protectrice 27 en définissant ainsi une chambre 54 de diffusion à l'intérieur de ladite coque protectrice 27 et la paroi de sommet 29, en obtenant ainsi une amélioration de la circulation de l'air et avantageusement de la surface d'échange à l'intérieur de la coque protectrice 27, résultant en une augmentation de la diffusion des produits contenus dans le réservoir 2.

Selon une caractéristique particulière de l'invention, la chambre de diffusion 54 communique avec l'orifice axial 52 du moyen absorbant 35 par un passage 53. Le moyen absorbant 35 étant disposé à un niveau intermédiaire, une chambre inférieure 56 est également définie à la partie inférieure de la coque protectrice 27. On conçoit ainsi que l'air entrant par les ouvertures 31 passe dans la chambre 56, constituant une chambre de répartition d'air, passe au travers du tampon absorbant 35 et également dans l'orifice axial 53 et aboutit dans la chambre 54 de diffusion avant de sortir de la coque protectrice 27 par les orifices 50. Ceci permet ainsi d'augmenter la circulation d'air et également la surface d'échange du matériau absorbant 35 avec l'air.

Pour maintenir en position intermédiaire le moyen absorbant 35, on prévoit avantageusement des moyens d'arrêt 58, 24 du moyen absorbant 35 en position intermédiaire. Ces moyens d'arrêt peuvent se subdiviser en premiers moyens d'arrêt 58 contre un déplacement vers le haut du moyen absorbant 35 et/ou en des seconds moyens d'arrêt 24 du moyen absorbant 35 contre un déplacement vers le bas. Les premiers moyens d'arrêt 58 contre un déplacement vers le haut peuvent comprendre un épaulement annulaire radialement saillant vers l'intérieur de la coque protectrice 27, comme représenté.

Ces premiers moyens d'arrêt contre un déplacement vers le haut du moyen absorbant 35 peuvent également comprendre une extension des moyens d'actionnement 39-40 du dispositif d'éjection 11.

En effet, selon l'invention, les moyens d'actionnement 39, 40 comprennent avantageusement un ergot 40 solidaire de la partie convexe 39 opposée à la partie concave 36 de l'extrémité supérieure 29 formant paroi de sommet, qui est d'une longueur suffisante pour que sa face inférieure 41 soit située juste au-dessus du dispositif d'éjection 11 formant bouton-poussoir classique, dans sa partie incurvée. On peut prévoir des extensions 43 venant se positionner latéralement relativement au dispositif d'éjection 11.

Cet ergot 41 peut être formé par deux cloisons disposées perpendiculairement entre elles. Ce sont ces cloisons qui portent les extensions 43. Dans une variante de réalisation, on peut prévoir ces cloisons d'une plus grande dimension de manière à avoir un empattement supérieur au diamètre de l'orifice axial 52, comme montré en traits mixtes en 40′. Ces cloisons du l'ergot 40′ viendront s'appliquer par leurs extensions 43 au-dessus du tampon absorbant 35 et constitueront ainsi des moyens d'arrêt contre un déplacement vers le haut.

On comprend également que, selon l'invention, la combinaison des moyens 36-39-40 constitue une zone déformable de l'extrémité supérieure 29 de la coque protectrice 27 qui est avantageusement située au-dessus du dispositif d'éjection 11, l'ergot 40 constituant une partie intégrante avec cette zone déformable du moyen d'actionnement de l'extérieur du dispositif d'éjection 11.

Selon une variante de réalisation particulièrement avantageuse de l'invention, le capuchon protecteur 15 est formé d'une première partie 16 fixée de manière réversible ou non sur la partie supérieure 3 du réservoir 2, et d'une deuxième partie constituée par la coque protectrice 27 qui est accouplable de préférence de manière réversible à cette première partie 16 en pouvant de préférence pivoter autour de celle-ci.

La première partie 16 est avantageusement en forme de bague surmontée d'une paroi 24 de forme cylindrique présentant des ouvertures 26 à intervalles réguliers. La coque protectrice est fixée sur la bague 16 de manière que la zone inférieure de la coque protectrice 30 soit emmanchée sur la paroi 24 et également de manière que les ouvertures 26 et 31 respectivement de la paroi 24 et de la zone inférieure 30 puissent être amenées en coïncidence ou se superposer par rotation de la coque protectrice 27 autour de la bague 16.

Avantageusement, les fixations de la première partie 16 de la coque protectrice 27 sont assurées par encliquetage. Pour ce faire, la base 30 de la coque protectrice 27 est prolongée par une couronne externe 32 munie au bord et à l'intérieur d'une nervure 33 circulaire. La jonction entre la couronne 32 et la zone 30 définit une surface d'appui 34.

Par ailleurs, la première partie 16 est formée d'un col 17 emmanché autour de la coupelle 14 puis d'un corps 18 s'évasant de manière à épouser, sans la toucher, la partie supérieure 3 du réservoir. L'extrémité de cette partie évasée est de forme cylindrique. Elle comprend une première zone 21 cylindrique comportant une gouttière extérieure 19 puis à proximité un méplat 20 s'étendant vers l'extérieur formant surface d'appui pour la couronne externe 32 de la coque protectrice 27. Ensuite, une seconde zone cylindrique 22 est prévue de diamètre supérieur à la zone 21, comportant un rebord intérieur 23 assurant la fixation de la première partie 16 sur les bords 8 de la coupelle 7.

En outre, cette première partie 16 est surmontée d'une couronne cylindrique 24, d'un diamètre légèrement plus petit que le diamètre de la coque protectrice 27, solidarisé au méplat 20 du corps 18. Cette couronne 24 pénètre à l'intérieur de la coque protectrique 27 lorsque celle-ci est encliquetée sur la première partie 16. Ainsi, cette couronne cylindrique 24 est pourvue d'ouvertures 26 régulièrement espacées de section identique à celle des ouvertures 31 de la coque protectrice 27. Par exemple, ces ouvertures peuvent être de section rectangulaire, afin d'offrir une grande section d'entrée à l'air.

On conçoit que la rotation de la coque protectrice 27 autour de la première partie 16 rende possible de fermer complètement les ouvertures 31, ou de régler le degré de superposition des ouvertures 26 et 31, ce qui permet de régler le débit de diffusion, ce débit de diffusion étant encore fonction de la quantité initiale de produits pulvérisés dans le tampon absorbant 25. On comprend lors d'une absence totale de superposition des ouvertures 26 et 31 comme représenté aux figures 3 et 4 sur les demi-vues de gauche, la diffusion soit arrêtée.

Avantageusement, la force de maintien de la première partie 16 à la partie supérieure 3 du réservoir 2 est supérieure à la force de maintien de la coque protectrice 27 sur la première partie 16.

En référence aux figures 5 et 6, on a représenté une variante de réalisation selon laquelle une ouverture 44 est pratiquée dans la coque protectrice 28 de manière que cette ouverture soit, lorsque le capuchon protecteur 15 est positionné sur le réservoir 2, à hauteur du cône d'éjection 47. Le tampon 45 est naturellement interrompu en 46 de manière à permettre le passage de la matière éjectée jusqu'à l'ouverture. Ainsi, dans cette variante il est possible, sans enlever la coque protectrice 28, d'effectuer les deux opérations par simple rotation de celle-ci, d'une part pour que le cône d'éjection soit dirigé sur le tampon 45 (position de diffusion), d'autre part pour que le cône d'éjection soit dirigé vers l'ouverture 44 (position d'éjection).

Le fonctionnement du capuchon protecteur selon l'invention est le suivant, en référence aux figures 1 à 4.

Au repos, la coque protectrice 28 est fixée sur la partie supérieure 3 du réservoir de stockage 2 sans être en contact avec le dispositif d'éjection 11. Quand on veut pulvériser du produit actif directement dans l'atmosphère, on ôte la coque protectrice 27 de la première partie 16 qui reste fixée à la partie supérieure du réservoir du fait de la force supérieure de maintien et on appuie directement sur le dispositif d'éjection 11, ce qui entraîne l'éjection-distribution d'une giclée de produits contenus dans le réservoir de stockage 2 (gaz vecteur + produit actif tel que parfums et/ou déodorants et/ou insecticides). Quand on a fini cette opération, on referme l'ensemble de l'appareil en fixant ou en cliquetant à nouveau le capuchon protecteur 27 ou coque sur le réservoir de stockage 2 formant bombe aérosol.

Ce dispositif selon l'invention peut également fonctionner en diffuseur et pour cela, il suffit alors d'appuyer sur la zone souple 29, ce qui va agir sur le dispositif d'éjection 11 par l'intermédiaire de l'ergot 40 et entraîner une éjection du contenu du réservoir hors du dispositif d'éjection 11, en direction de l'élément absorbant 35 qui absorbe sensiblement la totalité de ce qui a été éjecté.

On laisse alors l'ensemble de l'appareil au repos.

Les produits absorbés sont réémis lentement, d'eux-mêmes, hors de l'élément tampon absorbant 35, d'abord dans la chambre de diffusion 54 puis, lorsque l'on fait faire une rotation à la coque protectrice 27 de manière que les ouvertures 31 de la zone 30 soient partiellement ou totalement superposées aux ouvertures 26 de la couronne 24 de la première partie 16, les produits absorbés diffusent hors de la coque protectrice 27 en direction et dans l'atmosphère environnante (position représentée aux figures 3 et 4 aux demi-vues de droite).

Comme précédemment indiqué, le débit de diffusion est fonction du degré de superposition des ouvertures 26 et 31 et évidemment de la quantité initiale de produits pulvérisés dans le tampon.

La diffusion est arrêtée aisément en faisant tourner la coque protectrice 27 de manière qu'il n'y ait plus de superposition des ouvertures 26 et 31 comme représenté aux figures 3 et 4 aux demi-vues de gauche).

Selon une caractéristique particulièrement intéressante de l'invention, les ouvertures (prises comme la combinaison des ouvertures 26 et 31) constituent généralement 1 à 20 % de la surface totale extérieure du capuchon protecteur 15, de préférence 2 à 5 % ; pratiquement cela conduit souvent à des surfaces d'ouvertures de 1 à 10 cm², de préférence 2 à 5 cm². La forme et la position des ouvertures 26, 31 sont très variables et ont peut d'importance dans la mesure où elles se situent dans la partie inférieure de la coque protectrice 27, en-dessous du tampon absorbant 35.

Bien que la nature du matériau absorbant 35 selon l'invention ne soit pas déterminée étroitement, on peut cependant, à titre non limitatif citer des matériaux tels que :
- les composés minéraux poreux obtenus par exemple par frittage, compression ou cuisson de poudre de fibres ;
- les composés organiques, naturels ou synthétiques, tels que la cellulose, les composés polyacryliques, les polyesters, les polyéthers, les polyruéthannes, les polyoléfines, les polyamides.

Plus généralement, à titre de matériaux absorbants 35, on peut utiliser tous composés susceptibles d'avoir des propriétés absorbantes, notamment ceux en forme de fibres (textile, non tissé, des bourres), ou de mousse ou autres masses poreuses avantageusement ayant des pores ou cellules ouvertes.

Le volume du milieu absorbant est généralement compris entre 4 et 50 % du volume intérieur du bouchon protecteur, de préférence entre 10 et 40 %. La capacité d'absorption du matériau 35 est généralement suffisante pour absorber 1 à 10 g de produit issu du dispositif d'éjection.

Grâce à l'invention, on améliore de manière radicale la circulation de l'air à l'intérieur du dispositif protecteur 15, ainsi que la surface d'échange, ceci permet d'utiliser de très faibles quantités de produits actifs, en permettant l'utilisation de produits secs, c'est-à-dire sans liquide mouillant. On peut par exemple utiliser un parfum à 1 % dans du gaz vecteur comme par exemple du butane.

Naturellement, l'invention comprend tous les moyens constituant des équivalents techniques des moyens décrits sans sortir du cadre des revendications.

## Revendications

1. Dispositif formant capuchon protecteur (15) d'un dispositif de stockage et de pulvérisation (2) de produits destinés à être distribués dans l'atmosphère, tels que par exemple parfums et déodorants, habituellement communément appelés bombe aérosol, constitués d'un réservoir de stockage (2) proprement dit des produits destinés à être distribués dans l'atmosphère, d'un dispositif d'éjection (10, 11) des produits, ledit capuchon (15) étant formé d'une coque protectrice (27) pour le dispositif d'éjection présentant une paroi latérale (28), par exemple sensiblement cylindrique, ayant une extrémité inférieure (30-32) destinée à venir coopérer avec le réservoir de stockage (2), ainsi qu'une extrémité supérieure (29) sensiblement fermée constituant une paroi de sommet (36-37-38) de la coque protectrice (27) ; un moyen (35) absorbant le produit, essentiellement en forme de disque et s'étendant jusqu'à la paroi latérale (28), étant disposé transversalement à l'intérieur de la coque protectrice (27) permettant l'absorption et la réémission dudit produit dans l'atmosphère ; des moyens de diffusion (31, 50) étant prévus pour permettre une diffusion dudit produit à l'extérieur de la coque protectrice (27), ces moyens de diffusion comprenant une ou plusieurs ouvertures (31) prévues dans la paroi latérale (28) de la coque protectrice (27) au niveau de l'extrémité inférieure (30), ainsi que un ou plusieurs orifices (50) au niveau de l'extrémité supérieure (29), pour créer une circulation d'air à l'extérieur de la coque protectrice et à travers le moyen absorbant, et un moyen (39-40) permettant l'actionnement du dispositif d'éjection (11) de l'extérieur du capuchon, caractérisé en ce que le moyen absorbant (35) en forme de disque est disposé à un niveau intermédiaire entre l'extrémité inférieure et l'extrémité supérieure de la coque protectrice (27) à l'intérieur de la coque protectrice (27), à distance de la paroi de sommet (29) de la coque protectrice (27) en définissant ainsi une chambre (54) de diffusion à l'intérieur de la coque protectrice entre le moyen absorbant (35) et la paroi de sommet (29), en obtenant ainsi une amélioration de la circulation de l'air et de la surface d'échange à l'intérieur de la coque protectrice (27), résultant en une amélioration de la diffusion desdits produits.

2. Dispositif formant capuchon protecteur selon la revendication 1, caractérisé en ce que le moyen absorbant (35) comprend un orifice axial (52) permettant le libre passage, avec jeu, du dispositif d'éjection (11), ledit dispositif d'éjection (11) se trouvant en regard dudit moyen absorbant (35) au moins pour certaines positions du capuchon (27) monté sur le réservoir ; et en ce que la chambre de diffusion (54) communique avec l'orifice axial (52) du moyen absorbant (35).

3. Dispositif formant capuchon protecteur selon la revendication 1 ou 2, caractérisé en ce que le moyen (39, 40) permettant d'actionner, de l'extérieur de la coque protectrice le dispositif d'éjection (11) comprend une zone déformable (36) de la coque protectrice.

4. Dispositif formant capuchon protecteur selon la revendication 3, caractérisé en ce que la zone déformable (36) est située au-dessus du dispositif d'éjection (11) et comprend un prolongement axial (40) vers le bas se terminant au-dessus du dispositif d'éjection (11), en position montée de la coque protectrice (27).

5. Dispositif formant capuchon protecteur selon une des revendications 1 à 4, caractérisé en ce qu'il comprend des moyens d'arrêt (58, 24) du moyen absorbant (35) en position intermédiaire.

6. Dispositif formant capuchon protecteur selon l'une des revendications 1 à 5, caractérisé en ce qu'il est formé d'une première partie (16) fixée de manière réversible ou non sur la partie supérieure (3) du réservoir (2), la coque protectrice (27) étant accouplable de préférence de manière réversible à cette première partie (16) et pouvant de préférence pivoter autour de celle-ci.

7. Dispositif formant capuchon protecteur selon la revendication 6, caractérisé en ce qu'il est formé d'une première partie (16) en forme de bague, surmontée d'une paroi (24) de forme cylindrique présentant des ouvertures (26) à intervalles réguliers, la coque protectrice est fixée sur la bague (16), de manière que la zone inférieure de la coque protectrice (30) soit emmanchée sur la paroi (24) et en ce que les ouvertures (26) et (31) respectivement de la paroi (24) et de la zone inférieure (30) puissent être amenées en coïncidence ou se superposer par rotation de la coque protectrice (27) autour de la bague (16).

8. Capuchon protecteur selon la revendication 6 ou 7, caractérisé en ce que les fixations de la première partie (16) et de la coque protectrice (27) sont assurées par encliquetage.

9. Dispositif formant capuchon protecteur selon une des revendications 6 à 8, caractérisé en ce que la force de maintien de la première partie (16) à la partie supérieure (3) du réservoir (2) est supérieure à la force de maintien de la coque protectrice (27) sur la première partie (16).

10. Dispositif formant capuchon protecteur selon une des revendications 5 à 9, caractérisé en ce que les moyens d'arrêt précités comprennent des premiers moyens d'arrêt (58) contre un déplacement vers le haut et/ou des seconds moyens d'arrêt (24) contre un déplacement vers le bas du tampon absorbant (35).

11. Dispositif formant capuchon protecteur selon la revendication 10, caractérisé en ce que les premiers moyens d'arrêt (58) contre un déplacement vers le haut comprennent un épaulement annulaire radialement saillant vers l'intérieur de la coque protectrice (27).

12. Dispositif formant capuchon protecteur selon la revendication 10, caractérisé en ce que les premiers moyens d'arrêt contre un déplacement vers le haut comprennent une extension du prolongement (40) axial vers le bas de la paroi de sommet (36-39), se terminant au-dessus du moyen absorbant (35).

13. Dispositif formant capuchon protecteur selon la revendication 10, 11 ou 12, caractérisé en ce que les seconds moyens d'arrêt précités contre un déplacement vers le bas sont constitués par la paroi (24) de la première partie (16).

14. Dispositif formant capuchon protecteur selon une des revendications 1 à 13, caractérisé en ce que la coque protectrice (27) est pourvue d'une ouverture (44) à hauteur du cône d'éjection, lorsque cette coque protectrice est positionnée sur le réservoir (2), et le moyen absorbant (35) est interrompu à ce niveau de manière à permettre le passage dudit cône.

15. Dispositif de stockage et de pulvérisation de produits destinés à être distribués dans l'atmosphère, constitué d'un réservoir de stockage (2), et d'un dispositif d'éjection (10, 11), caractérisé en ce qu'il est pourvu d'un dispositif formant capuchon protecteur (15) tel que défini selon l'une quelconque des revendications 1 à 14.

## Patentansprüche

1. Mit einer Schutzkappe (15) versehener Behälter (2) zum Versprühen von Produkten, die in die Atmosphäre abgegeben werden sollen, wie zum Beispiel Parfums und Deodorants, und der üblicherweise als Spraydose bezeichnet wird, der aus einem eigentlichen Behälter (2) für die Lagerung der in die Atmosphäre abzugebenden Produkte sowie aus einer Ausspritzvorrichtung (10, 11) für die Produkte besteht, wobei die Schutzkappe (15) aus einer Schutzglocke (27) für die Ausspritzvorrichtung gebildet ist, die eine zum Beispiel weitgehend zylindrische Seitenwand (28) aufweist, die ein unteres Ende (30, 32) enthält, das mit dem Lagerbehälter (2) zusammenwirkt, sowie ein weitgehend geschlossenes oberes Ende (29), das eine Oberwand (36, 37, 38) der Schutzglocke (27) bildet; weiterhin ein das Produkt absorbierendes Mittel (35), das im wesentlichen die Form einer Scheibe hat, die sich gegen die seitliche Wand (28) erstreckt, und das in Querrichtung im Inneren der Schutzglocke (27) angeordnet ist und die Absorption und die Wiederabgabe des Produktes in die Atmosphäre erlaubt; sowie Verteilermittel (31, 50) für die Verteilung des Produktes außerhalb der Schutzglocke (27), wobei diese Verteilermittel eine oder mehrere Öffnungen (31) aufweisen, die in der Seitenwand (28) der Schutzglocke (27) in Höhe des unteren Endes (30) vorgesehen sind, sowie eine oder mehrere Auslaßöffnungen (50) in Höhe des oberen Endes (29), um eine Zirkulation von Luft außerhalb der Schutzglocke und durch das absorbierende Mittel zu erzeugen, sowie ein Mittel (39, 40) für die Betätigung der Ausspritzvorrichtung (11) von der Außenseite der Schutzkappe,
**dadurch gekennzeichnet, daß** das absorbierende Mittel (35) in Form einer Scheibe auf einer Zwischenebene zwischen dem unteren Ende und dem oberen Ende der Schutzglocke (27) innerhalb der Schutzglocke (27) im Abstand von der Oberwand (29) der Schutzglocke (27) angeordnet ist und so eine Verteilerkammer innerhalb der Schutzglocke zwischen dem absorbierenden Mittel (35) und der Oberwand (29) bildet, wodurch eine Verbesserung der Zirkulation von Lut und der Austauschfläche innerhalb der Schutzglocke (27) erreicht wird, woraus sich eine verbesserte Verteilung der Produkte ergibt.

2. Eine Schutzkappe nach Anspruch 1,
**dadurch gekennzeichnet, daß** das absorbierende Mittel (35) eine axiale Auslaßöffnung (52) aufweist, die den freien Durchgang mit Spiel der Ausspritzvorrichtung (11) ermöglicht, wobei sich die Ausspritzvorrichtung (11) mindestens in einigen Positionen der auf den Behälter aufgesetzten Schutzkappe (27) gegenüber dem absorbierenden Mittel (35) befindet, und dadurch daß die Verteilerkammer (54) mit der axialen Auslaßöffnung (52) des absorbiernden Mittels (35) kommuniziert.

3. Eine Schutzkappe nach einem der Ansprüche 1 oder 2, **dadurch gekennzeichnet, daß** das Mittel (39, 40) für die Betätigung der Ausspritzvorrichtung (11) von der Außenseite der Schutzglocke einen verformbaren Bereich (36) der Schutzglocke aufweist.

4. Eine Schutzkappe nach Anspruch 3,
**dadurch gekennzeichnet, daß** der verformbare Bereich (36) oberhalb der Ausspritzvorrichtung (11) angeordnet ist und eine axiale Verlängerung (40) nach unten aufweist, die in montierter Stellung der Schutzglocke (27) oberhalb der Ausspritzvorrichtung (11) endet.

5. Eine Schutzkappe nach einem der Ansprüche 1 bis 4,
**dadurch gekennzeichnet, daß** sie Mittel (58, 24) für die Arretierung des absorbierenden Mittels (35) in Zwischenstellung aufweist.

6. Eine Schutzkappe nach einem der Ansprüche 1 bis 5,
**dadurch gekennzeichnet, daß** sie aus einem ersten Teil (16) besteht, der umkehrbar oder nicht umkehrbar an dem oberen Teil (3) des Behälters (2) befestigt ist, wobei die Schutzglocke (27) vorzugsweie umkehrbar mit diesem ersten Teil (16) gekoppelt ist und sich vorzugsweise um diesen drehen kann.

7. Eine Schutzkappe nach Anspruch 6,
**dadurch gekennzeichnet, daß** sie aus einem ersten Teil (16) in Form eines Ringes besteht, auf dem eine zylindrische Wand (24) sitzt, die in regelmäßigen Abständen Öffnungen (26) aufweist, und die Schutzglocke auf dem Ring (16) so befestigt ist, daß der untere Bereich der Schutzglocke (30) auf die Wand (24) aufgesteckt ist, und dadurch daß die jeweiligen Öffnungen (26) und (31) der Wand (24) und des unteren Bereiches (30) durch Drehen der Schutzglocke (27) um den Ring (16) übereinandergeschoben werden können.

8. Eine Schutzkappe nach einem der Ansprüche 6 oder 7,
**dadurch gekennzeichnet, daß** die Befestigung des ersten Teils (16) und der Schutzglocke (27) durch Verklinken erfolgt.

9. Eine Schutzkappe nach einem der Ansprüche 6 bis 8,
**dadurch gekennzeichnet, daß** die kraft der Halterung des ersten Teils (16) an dem oberen Teil (3) des Behälters (2) größer ist als die kraft der Halterung der Schutzglocke (27) an dem ersten Teil (16).

10. Schutzkappe nach einem der Ansprüche 5 bis 9,
**dadurch gekennzeichnet, daß** die vorgenannten Arretierungsmittel erste Mittel (58) für eine Arretierung gegen eine Bewegung des absorbierenden Puffers (35) nach oben und/oder zweite Mittel (24) für eine Arretierung gegen eine Bewegung des absorbierenden Puffers (35) nach unten enthalten.

11. Eine Schutzkappe nach Anspruch 10,
**dadurch gekennzeichnet, daß** die ersten Mittel (58) für eine Arretierung gegen eine Bewegung nach oben eine radial nach innen in die Schutzglocke (27) ragende kreisförmige Schulter enthalten.

12. Eine Schutzkappe nach Anspruch 10,
**dadurch gekennzeichnet, daß** die ersten Mittel für eine Arretierung gegen eine Bewegung nach oben eine Ausweitung der axialen Verlängerung (40) nach unten der Oberwand (36, 39) aufweisen, die oberhalb des absorbierenden Mittels (35) endet.

13. Eine Schutzkappe nach einem der Ansprüche 10, 11 oder 12,
**dadurch gekennzeichnet, daß** die zweiten vorgenannten Mittel für eine Arretierung gegen eine Bewegung nach unten aus der Wand (24) des ersten Teils (16) gebildet werden.

14. Eine Schutzkappe nach einem der Ansprüche 1 bis 13,
**dadurch gekennzeichnet, daß** die Schutzglocke (27) mit einer Öffnung (44) in Höhe des Ausspritzkonus versehen ist, wenn die Schutzglocke auf dem Behälter (2) positioniert ist und daß das absorbierende Mittel (35) an dieser Stelle unterbrochen ist, um den Durchgang des Konus zu ermöglichen.

15. Vorrichtung für die Lagerung und das Versprühen von Produkten, die in die Atmosphäre abgegeben werden sollen, bestehend aus einem Behälter (2) und einer Ausspritzvorrichtung (10, 11),
**dadurch gekennzeichnet, daß** sie mit einer Vorrichtung versehen ist, die eine Schutzkappe (15) bildet, wie sie in einem der Ansprüche 1 bis 14 definiert ist.

## Claims

1. Device forming a protective cap (15) for a device (2) for storing and atomising products intended to be dispensed into the atmosphere, such as for example perfumes and deodorants, normally and commonly referred to as an aerosol can, consisting of the storage reservoir (2) itself for the product intended to be dispensed into the atmosphere, a device (10, 11) for ejecting the products, the said cap (15) being formed by a protective shell (27) for the injection device having a lateral wall (28), for example substantially cylindrical, having a bottom end (30-32) intended to interact with the storage reservoir (2), and a substantially closed top end (29) constituting a top wall (36-37-38) of the protective shell (27); means (35) absorbing the product, essentially in the form of a disk and extending as far as the lateral wall (28), being disposed transversely inside the protective shell (27) enabling the said product to be absorbed and re-emitted into the atmosphere; diffusion means (31, 50) being provided to allow diffusion of the said product outside the protective shell (27), these diffusion means comprising one or more openings (31) provided in the lateral wall (28) of the protective shell (27) at the bottom end (30), together with one or more orifices (50) at the top end (29), to create circulation of air outside the protective shell and through the absorbent means, and means (39-40) permitting the actuation of the ejection device (11) outside the cap, characterised in that the absorbent means (35) in the form of a disk is disposed at an intermediate level between the bottom end and top end of the protective shell (27) inside the protective shell (27), at a distance from the top wall (29) of the protective shell (27), thus defining a diffusion chamber (54) inside the protective shell between the absorbent means (35) and the top wall (29), thus obtaining an improvement in the circulation of air and in the exchange surface inside the protective shell (27), resulting in an improvement in the diffusion of the said products.

2. Device forming a protective cap according to Claim 1, characterised in the absorbent means (35) comprises an axial orifice (52) allowing the free passage, with clearance, of the ejection device (11), the said ejection device (11) being located opposite the said absorbent means (35) at least for certain positions of the cap (27) mounted on the reservoir; and in that the diffusion chamber (54) communicates with the axial orifice (52) in the absorbent means (35).

3. Device forming a protective cap according to Claim 1 or 2, characterised in that the means (39, 40) making it possible to actuate the ejection device (11) from outside the protective shell includes a deformable area (36) of the protective shell.

4. Device forming a protective cap according to Claim 3, characterised in that the deformable area (36) is situated above the ejection device (11) and comprises an axial extension (40) downwards terminating above the ejection device (11), when the protective shell (27) is in its fitted position.

5. Device forming a protective cap according to one of Claims 1 to 4, characterised in that it comprises means (58, 24) for arresting the absorbent means (35) in an intermediate position.

6. Device forming a protective cap according to one of Claims 1 to 5, characterised in that it is formed by a first part (16) fixed reversibly or otherwise to the top part (3) of the reservoir (2), the protective shell (27) being connectable, preferably reversibly, to this first part (16) and preferably being able to pivot about the latter.

7. Device forming a protective cap according to Claim 6, characterised in that it is formed by a first part (16) in the form of a ring, surmounted by a cylindrically shaped wall (24) having openings (26) at regular intervals, the protective shell is fixed to the ring (16), so that the bottom area of the protective shell (30) is fitted onto the wall (24) and in that the openings (26) and (31) respectively in the wall (24) and the bottom area (30) can be made to coincide or be superimposed by rotation of the protective shell (27) about the ring (16).

8. Protective cap according to Claim 6 or 7, characterised in that the first part (16) and the protective shell (27) are fixed by snapping-on.

9. Device forming a protective cap according to one of Claims 6 to 8, characterised in that the force for holding the first part (16) on the top part (3) of the reservoir (2) is greater than the force for holding the protective shell (27) on the first part (16).

10. Device forming a protective cap according to one of Claims 5 to 9, characterised in that the aforesaid arresting means comprise first means (58) of arresting against an upward movement and/or second means (24) of arresting against a downward movement of the absorbent plug (35).

11. Device forming a protective cap according to Claim 10, characterised in that the first means of arresting (58) against an upward movement comprise an annular collar projecting radially towards the inside of the protective shell (27).

12. Device forming a protective cap according to Claim 10, characterised in that the first means of arresting against an upward movement comprise an extension of the downward axial extension (40) of the top wall (36-39), terminating above the absorbent means (35).

13. Device forming a protective cap according to Claim 10, 11 or 12, characterised in that the aforesaid second means of arresting against a downward movement are formed by the wall (24) of the first part (16).

14. Device forming a protective cap according to one of Claims 1 to 13, characterised in that the protective shell (27) has an opening (44) level with the ejection cone, when this protective shell is positioned on the reservoir (2), and the absorbent means (35) is interrupted at this level so as to allow passage of the said cone.

15. Device for storing and atomising products intended to be dispensed into the atmosphere, consisting of a storage reservoir (2) and an ejection device (10, 11), characterised in that it has a device forming a protective cap (15) as defined according to any one of Claims 1 to 14.
